# EUROPEAN PATENT APPLICATION

(11) **EP 1 779 883 A1**
(43) Date of publication of application: **02.05.2007**
(21) Application number: 05077469.4
(22) Date of filing: 27.10.2005
(51) Int. Cl.: A61M 5/44

(54) **Portable device for heating intravenous fluids**

(71) Applicant: Hotwatch ApS, 8000 Arhus C (DK)
(72) Inventor: Vest, Jan Erik, 8000 Arhus C (DK)
(74) Representative: Schmidt, Jens Joergen

(57) **Abstract**

A system is disclosed for heating a flow of intravenous fluid comprising an intravenous tube (3) for connecting a source of intravenous fluid to an intravenous catheter, the intravenous tube comprising heating means (11) integrated therein for raising the temperature of the intravenous flow in the tube and connection means (6) for connecting said heating means with an external energy source, and an external unit (2) comprising the external energy source and activating means for activating the heating means in the intravenous tube by providing electrical or therrnal power to the heating means.
By providing integrated heating means in the tube, the path between a source of intravenous fluid and an intravenous catheter is ensured to be highly hygienic, because the number of connections wherein the flow of intravenous fluid can be contaminated is limited to a minimum as compared to other systems with multiple connections to external units.

## Description

### FIELD OF INVENTION

The present invention relates to a system for heating a flow of intravenous fluid and to an assembly for a system for connecting a source of intravenous fluid to a patient.

### BACKGROUND

Army medics, paramedics, mountain rescue teams and other types of rescue personnel are often in need for administering intravenous fluids to critically wounded persons. However, when these intravenous fluids are carried in the field the temperature of these fluids is often substantially lower than the body temperature of the patient. The injection of cold intravenous fluids in to a vein of a patient presents a substantial risk for hyperthermia, which poses a threat to the patient's life. Therefore there is a need for a portable device to heat the intravenous fluid to a temperature closer to the body temperature before it enters the patient's veins. Further there is a need for controlling the amount of intravenous fluid delivered to a patient.

WO 01/62194 A1 discloses a portable disposable intravenous fluid warming system. The system comprises a battery powered unit with an integrated heating element having an inlet and an outlet for intravenous fluids, sized to fit standard intravenous line connectors. The inlet of the unit is connected to an intravenous bag and the outlet is connected to an intravenous catheter via standard intravenous tubes.

US 6,236,809 discloses a system for heating intravenous fluids comprising a disposable heat exchanger with an inlet and an outlet for intravenous fluids and a portable battery powered unit including a heating element and temperature controller. The inlet of the disposable heat exchanger comprises a fitting for connection to a source of intravenous fluid via a first length of tubing, and the outlet of the disposable heat exchanger likewise comprises a fitting for connection to an intravenous catheter via a second length of tubing.

US 5,250,032 disclose yet another system for warming intravenous fluids. The system comprises a battery powered heating element and a control circuit placed in housing mounted on the patients arm. An elongated portion of a standard intravenous tube is inserted in a channel in the heating element, whereby a flow of intravenous fluid in the intravenous tube is heated

US 3,908,652 disclose a medical infusion apparatus having an infusion flask exchangeably fastened to a casing, an infusion tube provided with a drip chamber, an electrical control system, and a drip valve or an infusion pump. A heat radiation source may be incorporated in the apparatus in order to heat the infusion liquid on its path from the flask to the infusion tube.

It is an object of the present invention to provide a simple inexpensive system to heat a flow of intravenous fluid prior to its entry into the vein of a patient, and to heat said flow of intravenous fluid sufficiently to avoid hyperthermia to the body of a receiving patient.

Additionally, it is an object of the present invention to provide a hygienic system for heating a flow of intravenous fluid between a source of intravenous fluid and an intravenous catheter.

It is further an object of the present invention to gentle heat the intravenous fluid in order to avoid damage to intravenous fluids such as blood.

It is also an object of the present invention to provide a system for accurately administering a flow of intravenous fluid to a patient.

### BRIEF DESCRIPTION OF THE PRESENT INVENTION

The invention provides a system for heating a flow of intravenous fluid comprising an intravenous tube having a liquid inlet and a liquid outlet for connecting a source of intravenous fluid to an intravenous catheter, said intravenous tube comprising heating means integrated therein for raising the temperature of the intravenous flow in the tube, and connection means for connecting said heating means with an external energy source, and an external unit comprising the external energy source and activating means for activating the heating means in the intravenous tube.

By integrating the heating means in the intravenous tube, a single infrangible path between a source of intravenous fluid and an intravenous catheter is obtained, wherein a flow of intravenous fluid is heated. Hereby, a simple portable system is provided which requires only two connections to external units as compared to most known systems which require multiple connections between several external units, i.e. that a separate heating element is not required by the provision of the system according to present invention to be inserted in between two lengths of intravenous tubing.

The simplicity of the present invention further eases the process of administering intravenous fluids to critical wounded persons and minimises the risk for rescue personnel under stress, e.g. an army medic at the front line or a paramedic at a large scene of accident, form making mistakes during the infusion of heated intravenous fluids, in that a separate heating element is not required to be connected to the flow path from the source of intravenous fluid to the patient.

Further the single infrangible path between a source of intravenous fluid and an intravenous catheter provides a highly hygienic system, because the number of connections wherein the flow of intravenous fluid can be contaminated is limited to a minimum as compared to other systems with multiple connections to external units.

By the term heating means is understood one or more electrical heating elements or a heat exchanger for exchanging heat from a heating source and to the flow of intravenous fluid. Generally, the surface area of said heating means in contact with the intravenous fluid is at least 20 cm², preferably at least 30 cm² and most preferred at least 40 cm². By having a substantially large heating area in contact with the intravenous fluid, a low contact temperature between the heating means and the intravenous fluid is obtained, thereby providing a gentle heating of the intravenous fluid.

The intravenous tube is preferably delivered sterile and enclosed in a sterile packaging until time of use. Hence, the risk of giving the patient an infection is substantially reduced.

In another aspect of the present invention said integrated heating means comprises one or more heating wires inlaid in the enclosure of the intravenous tube. The intravenous fluid is heated while flowing through the enclosure of the intravenous tube, thereby providing a large area of the heating means which is in contact with the intravenous fluid. This means that the intravenous fluid can be gently heated, thus preventing damage to fragile intravenous fluids such as blood.

In yet another aspect of the present invention said integrated heating means comprising heating wires encased in the wall of the intravenous tube. Hence, the heating wires are protected within the wall of the intravenous tube, thereby providing heating means that is resistant for use in harsh environments, such as when the intravenous tube are carried in the field in a densely packed bag which is exposed to large temperature gradients, shocks and blows.

The heating means extends preferably a substantial portion of the tube length preferably between 75 cm and 200 cm of the tube length such as between 125 cm and 175 cm of the tube length. Hence, it is assured that the flow of intravenous fluid is gently heated without being damaged, and that the heat loss through the tube wall is reduced to a minimum.

The energy source may comprise means for supplying electrical power to the heating means through said connection means. Hence, electrical power is provided for electrical heating means integrated within the intravenous tube. Alternatively, the external unit may comprise a heating element which in operation is in thermal contact with the heating means, in this case a heat exchanger, of the intravenous tube.

In another aspect of the present invention said connection means and said heating means constitutes a common unit forming a part of the intravenous tube and positioned in the vicinity of the liquid outlet of said intravenous tube. The external unit may in particular comprise an opening for insertion of the common unit, wherein said opening includes the activating means for supplying said common unit with energy. Thereby, energy is provided for the heating means integrated within the intravenous tube to heat the flow of intravenous fluid, when the common unit is inserted into the external unit.

By the term energy is understood either electrical energy or thermal energy. Thus, the activating means for supplying the common unit with energy may in one embodiment comprise electrical connection means, and said common unit comprises an electrical heating element. Hence, the heating element is in contact with the intravenous fluid thus providing an optimal heat transfer to the intravenous fluid.

Alternatively, said activating means comprises an electrical heating element formed in the external unit along a perimeter of said opening of said external unit, and said common unit comprises a heat exchanger designed to fit into said opening in the external unit. Thereby all electrical components of the system are enclosed within the same unit, thus providing a fairly simple system to use and maintain.

In another aspect of the present invention the system further comprises flow control means, such as a pump, for controlling the flow rate of intravenous fluid delivered to a patient. Thereby, a flow of intravenous fluid can be provided without the aid of gravity and the amount of intravenous fluid administered to a patient can easily be controlled by the rescue personnel. The flow control means may in particular comprise a peristaltic pump, i.e. a pump operating by manipulating the outer surface of a flexible length of the intravenous tube without having parts of the pump in directly contact with the fluid itself.. Thereby, the flow of intravenous fluid delivered to a patient can be controlled without undermining the sterile path of the intravenous fluid from a source of intravenous fluid to a patient. Further the amount of intravenous fluid delivered to the patient can easily be established and controlled.

In an aspect of the present invention said activating means comprises control means for controlling said heating means and/or said means for controlling the flow rate of intravenous fluid delivered to a patient according to at least one control input from means for providing a measure of at least one physical property of the flow of intravenous fluid. Hence, the activating means are enabled to provide a controlled heating of the intravenous fluid.

In a further aspect of the present invention said means for providing a measure of at least one physical property of the intravenous fluid comprises means for providing a measure of the flow rate of fluid in the intravenous tube and providing an input accordingly to the control means. This enables the control means to automatically monitor and control the flow rate of the intravenous fluid supplied to a patient.

In yet another aspect of the present invention said means for providing a measure of at least one physical property of the intravenous fluid comprises temperature measure means for measuring the temperature of the flow of fluid in the intravenous tube and providing an output accordingly to the control means. This enables the control means to automatically monitor and control the temperature of the intravenous fluid supplied to a patient.

In an aspect of the present invention said heating means are capable of raising the temperature of the intravenous fluid between 20°C and 30 °C preferably between 23 °C and 27 °C at a flow rate of 100 ml/min. Hereby it is assured that the temperature of the intravenous fluid is sufficiently high to avoid hyperthermia when injecting said intravenous fluid into a patient under most normal temperature conditions and at any possible flow rate.

In another aspect of the present invention, the system comprises means for limiting the temperature of the flow of intravenous fluid to a temperature interval between 35°C and 40°C such as between 36°C and 38°C independently of the flow rate of the intravenous fluid. Hence, it is assured that the heated intravenous fluid does not scorch the receiving patient.

In an aspect of the present invention said means for providing a measure of the flow rate of fluid in the intravenous tube comprises alarm means for providing an alarm signal when the source of intravenous fluid is empty. The alarm signal could be visual and/or audible, and would indicate to the rescue personnel that the source of intravenous fluid has to be replaced.

In a further aspect of the present invention said means for providing a measure of the flow rate of fluid in the intravenous tube comprises alarm means for providing an alarm signal when the flow of intravenous fluid is interrupted. Likewise the alarm signal could be visual and/or audible, and would indicate to the rescue personnel that the flow of intravenous fluid is interrupted e.g. because of a twisted or pinched intravenous tube, and thus that the flow of intravenous fluid has to be re-established.

In an aspect of the present invention said activating means comprises means for automatically transferring energy from said external energy source to said heating means via said connecting means when said connection means is connected to the energy source. Hence, the system will be activated automatically when connecting the heating means to the energy source, thereby adding to the simplicity of the system.

Moreover, the present invention relates to an assembly for connecting a source of intravenous fluid to a patient, the assembly comprising a sealed sterile packaging enclosing an intravenous tube having a liquid inlet and a liquid outlet for connecting said source of intravenous fluid to an intravenous catheter, said intravenous tube comprising heating means integrated therein for raising the temperature of the flow of intravenous fluid in the intravenous tube and connection means for connecting said heating means with an external energy source, wherein the mentioned items enclosed in the packaging are sterile. Thereby the risk of introducing an infection to the patient while administering intravenous fluids to the patient is substantially reduced.

The present invention also relates to heating means for heating a flow of intravenous fluid, comprising a length of a tube with a fluid connection defined therein, an electrical heating element integrated within said length of tube, and electrical connecting means for connecting said heating element to an electrical power source external to said heating means. Hereby is provided an intravenous tube wherein a flow of intravenous fluid can be gently heated to a temperature near body temperature prior to injecting it to a patient, thus avoiding hyperthermia or discomfort to the patient.

Furthermore, the present invention relates to a length of tube for heating liquid flowing therein comprising an inner opening with a substantially rectangular cross section, at least one sheet of electrical heating foil arranged in the longitudinal direction of said rectangular inner opening, connection means for connecting said electrical heating foil with an external energy source and/or external control means. By using sheets of electrical heating foil, a substantially large heating area in contact with the fluid is obtained, thereby providing a low contact temperature and thus a gentle heating of the fluid.

Advantageously the tube comprises two sheets of electrical heating foil arranged along the opposing longer sides of the rectangular inner portion. Hereby, a tube with a large heating area is obtained, which is capable of heating fluids at large flow rates and still providing a low contact temperature. Preferably said two sheets of electrical heating foil are short circuited at one end of the tube by a metal ring, and connected to a circuit at the other end for supplying said electrical heating foil with electrical power.

Advantageously the inner opening with a substantially rectangular cross section has a width in the range of 2 to 6 millimetres, preferably 3 to 5 millimetres, and a millimetres and a length of the longer sides in the range of 4 to 10 millimetres, preferably in the range of 5 to 8 millimetres. The width of the inner opening constitutes preferably in the range of 30% to 80%, preferably in the range of 40% to 65% of the length of the longer sides of the inner opening. Thus, the inner opening of the tube is in a preferred embodiment at least 2 millimetres wide and 4 millimetres long, more preferred at least 3 millimetres wide and 5 millimetres long..

### BRIEF DESCRIPTION OF THE DRAWINGS

The invention will be described in the following with reference to the drawings of which
Fig. 1 illustrates a system for heating a flow of intravenous fluid according to a preferred embodiment of the present invention,
Fig. 2 illustrates the external unit according to the embodiment of Fig. 1,
Fig. 3 illustrates an intravenous bag, a peristaltic pump and an intravenous tube according to a preferred embodiment the present invention,
Fig. 4 illustrates an intravenous bag and an alternative intravenous tube according to a second embodiment of the present invention, and
Fig. 5a to 5f illustrates cross sectional views of different embodiments of intravenous tubes according to the present invention.

### DETAILED DESCRIPTION OF THE PRESENT INVENTION

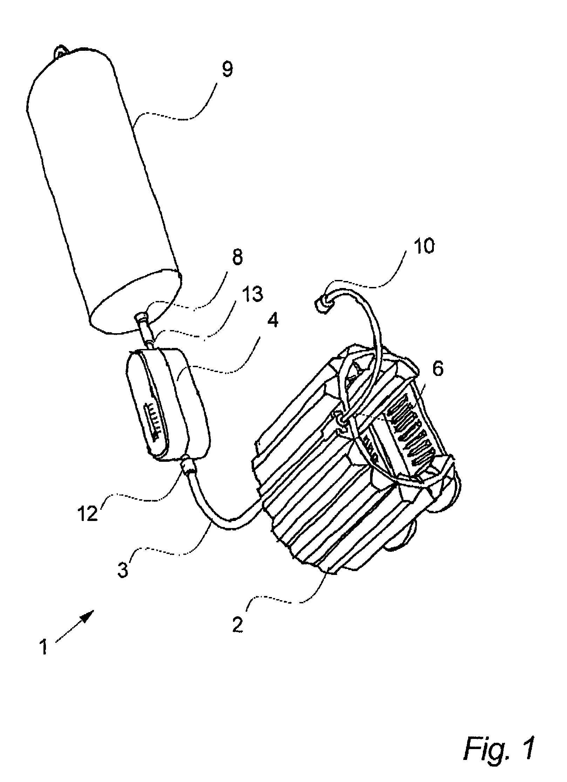

Fig. 1 illustrates a system 1 for heating a flow of intravenous fluid according to a preferred embodiment of the present invention. The system 1 comprises two separate main parts, an external unit 2 in form of a wristband and an intravenous tube 3 with electrical heating wires (examples shown in Figs. 5a to 5f) integrated therein. A peristaltic pump 4 is optionally mounted on the intravenous tube 3 for an accurate control of the intravenous fluid delivered to a patient.

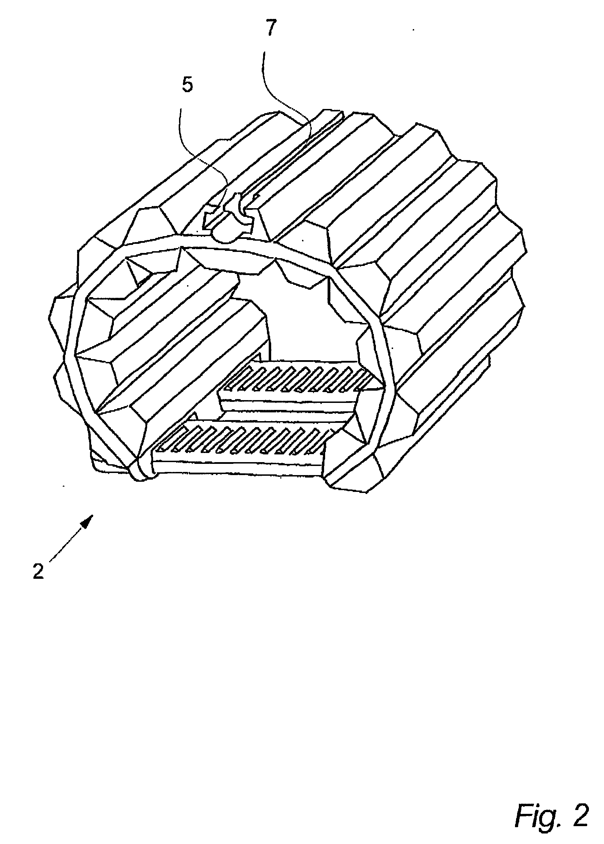

Fig. 2 illustrates the external unit 2 in detail. The external unit 2 comprises an energy source in form of a plurality of rechargeable batteries arranged along the circumference of the wristband. The external unit 2 further comprises a socket 5 sized to fit a corresponding connector 6 mounted on the intravenous tube 3. In extension of the socket 5 a channel 7 is arranged to support and secure a section of the intravenous tube 3 to the wristband. By forming the external unit 2 as a wristband, the intravenous tube 3 can be fixated to the wrist of a patient thereby preventing the intravenous catheter from being pulled out of the vein of the patient during transport.

The external unit 2 further comprises a temperature controller (not shown) in order to control the heating of the intravenous fluid. Inside the connector 6 a built-in temperature sensor (not shown), e.g. an RTD, provides an input to the temperature controller according to the actual temperature of the intravenous fluid. Hereby, the system is able to prevent that the intravenous fluid is heated above body temperature. As a further safety precaution the external unit 2 comprises a thermal fuse (not shown) arranged under the socket 5 in thermal contact with the intravenous fluid e.g. via the connector 6, to cut off the power to the electrical heating elements in case the temperature control system fails. Alternatively, the temperature sensor could also be arranged in the external unit 2 in thermal contact with the intravenous fluid e.g. via the connector 6. The connector 6 is preferably made of metal or a heat conductive plastic in order to provide the thermal fuse and/or the temperature sensor with an accurate measure of the temperature of the intravenous fluid.

The system is capable of raising the temperature of the intravenous fluid 25°C above its initial temperature at a flow rate of 100 ml/min. If the temperature of the intravenous fluid reaches body temperature (approximately 37°C) the temperature controller cuts off the power to the electrical heating wires until the temperature of the intravenous fluid is below body temperature.

The external unit 2 further comprises a second socket (not shown) for connecting the external unit 2 with a utility grid or the electrical system of a motorised vehicle, thereby providing means for supplying the electrical heating wires with an alternative source of electricity. Additionally the external unit 2 comprises means for recharging the rechargeable batteries, when the external unit is connected to an alternative source of electricity.

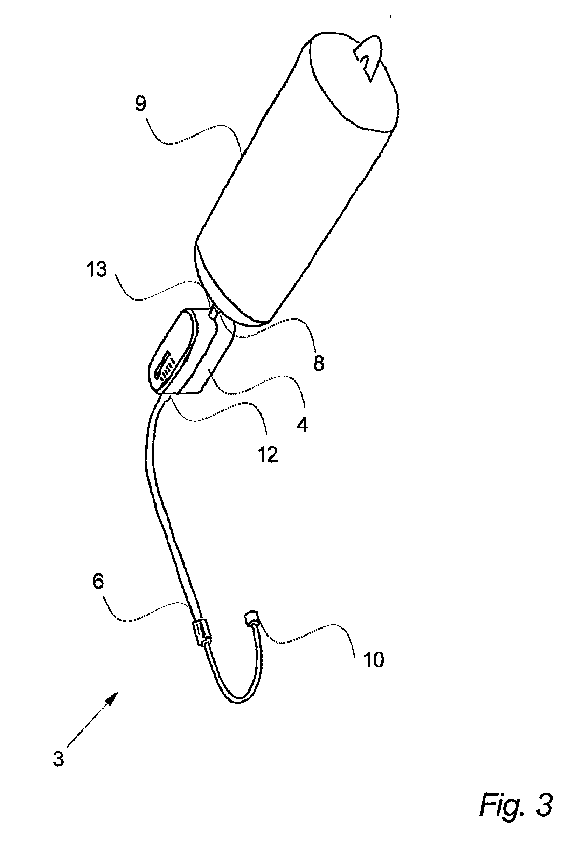

Fig. 3 illustrates a first preferred embodiment of the intravenous tube 3. The intravenous tube 3 comprises a liquid inlet 8 releasably connected to an intravenous bag 9 and a liquid outlet 10 for releasably connecting the intravenous tube 3 to an intravenous catheter (not shown) inserted into a vein of a patient. In order to heat a flow of intravenous fluid flowing through the intravenous tube 3, a pair of electrical heating wires 11 (examples shown in a cross sectional view in Figs. 5a to 5f) integrated within the intravenous tube 3 extends between the connector 6 and a metal ring 12. The electrical heating wires 11 are supplied with electrical power from the external unit 2 through the connector 6 and are short circuited by the metal ring 12.

A length of intravenous tubing 13 without integrated heating wires extends between the metal ring 12 and the liquid inlet 8. An optional peristaltic pump 4 can be mounted on the length of tubing 13 between the metal ring 12 and the liquid inlet 8 in order to accurately control the flow of intravenous fluid from the intravenous bag 9 to the intravenous catheter.

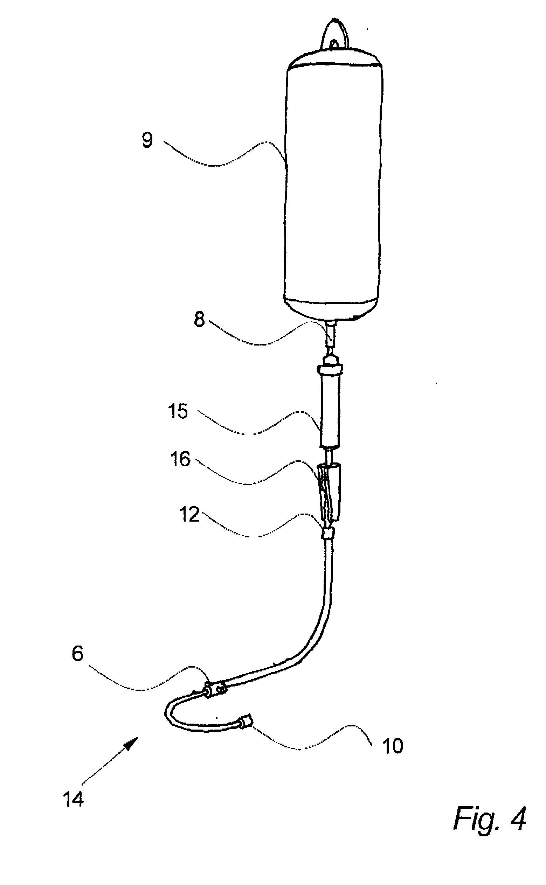

Fig. 4 illustrates an intravenous tube 14 according to an alternative embodiment of the present invention. As the intravenous tube 3 illustrated in Fig. 3 the alternative intravenous tube 14 comprises a connector 6, a liquid inlet 8, a liquid outlet 10 and integrated electrical heating wires 11 extending from the connector 6 to the metal ring 12 which short circuits the heating wires 11. The alterative intravenous tube 13 further comprises a drip chamber 15 and a clamp 16 as known from standard intravenous tubes. Thereby the flow of intravenous fluid can be controlled by the aid of gravity instead of the peristaltic pump 4.

Figs. 5a to 5f illustrates cross sectional views of different embodiments of the intravenous tube 3 with integrated electrical heating wires 11. Although the shape of the electrical heating wires 11 differs in the different embodiments shown in Figs. 5a to 5f, it is common to all embodiments that the electrical heating wires are short circuited by the metal ring 12. The embodiments shown in the figures are only meant as examples, so other embodiments with other configurations of the heating wires 11 are also within the scope of the present invention. Even though the examples are shown for the intravenous tube 3, the same configurations could be used for the heating wires integrated within the alternative intravenous tube 14.

Fig. 5a shows a cross sectional view of a first embodiment of the intravenous tube 3 according to the present invention. The intravenous fluid flows through a circular inner portion 17, and is heated via circular electrical heating wires 11 encased within the wall 18 of the intravenous tube 3.

Fig. 5b shows a cross sectional view of a second embodiment of the intravenous tube 3 according to present invention. The intravenous fluid flows through a circular inner portion 17, and is heated via circular heating wires 11 placed directly inside the circular portion 17 of the intravenous tube 3.

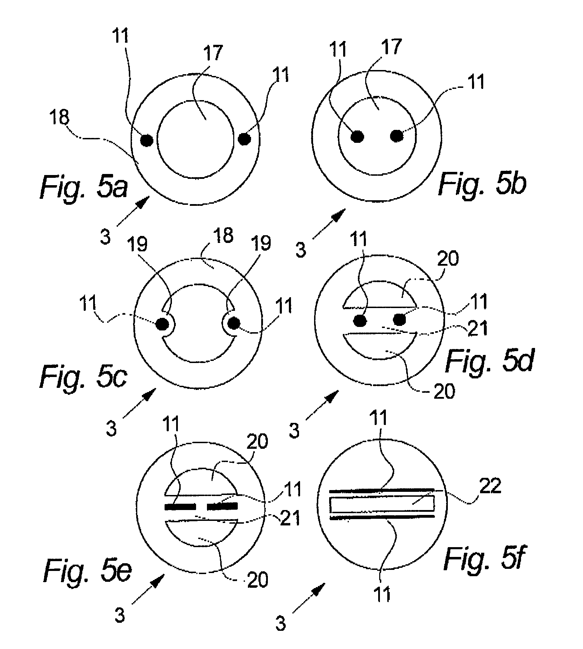

Fig 5c shows a cross sectional view of a third embodiment of the intravenous tube 3 according to present invention. The intravenous fluid flows through the circular inner portion 17 of the intravenous tube 3, and is heated by circular electrical heating wires 11 encased within internal thickenings 19 of the inner portion of the wall 18 of the intravenous tube 3.

Fig 5d shows a cross sectional view of a fourth embodiment of the intravenous tube 3 according to present invention. The intravenous fluid flows within two semi circular inner portions 20 of the intravenous tube 3 divided by an inner wall 21. The intravenous fluid is heated by circular electrical heating wires 11 encased within the inner wall 21.

Fig 5e shows a cross sectional view of a fifth embodiment of the intravenous tube 3 according to present invention. The intravenous fluid flows within two semi circular inner portions 20 of the intravenous tube 3 divided by an inner wall 21. The intravenous fluid is heated by rectangular electrical heating wires 11 encased within the inner wall 21. The rectangular shape of the electrical heating wires 11 provides a larger surface area from which heat can be transferred to the intravenous fluid, as compared to the circular electrical heating wires 11.

Fig 5f shows a cross sectional view of a preferred embodiment of the intravenous tube 3 according to present invention. The intravenous fluid flown through a rectangular inner portion 22 of the intravenous tube, and is heated by rectangular shaped heating wires 11 arranged along the longer sides of the rectangle. The electrical heating wires 11 could advantageously be two sheets of foil. Hereby the intravenous fluid flows past heating wires with a large surface area. Hence, an effective transfer of heat between the heating wires 11 and the intravenous fluid is provided, thereby minimising the heat loss from the intravenous fluid. Further the ratio between the area of the electrical heating element and the power delivered to the heating element can be substantially lowered, thus precluding a meltdown of the heating element. Another advantage of the low power to area ratio is a low contact temperature between the heating wires and the intravenous fluid, thereby providing a gentle heating of the intravenous fluid.

Now returning to the embodiment shown in Fig. 1. When the connector 6 of the intravenous 3 tube is inserted into the socket 5 of the external unit 2, electrical power is automatically transferred from batteries in the external unit 2 to the electrical heating wires in the intravenous tube 3. The electrical heating wires 11 with an outer diameter of 0.5 millimetres extend approximately 150 cm from the connector 6 to the metal ring 12. Thereby a gentle heating of the flow of intravenous fluid is assured and damage to fragile intravenous fluids such as blood is prevented. Further heat loss from the flow of intravenous fluid when flowing through the intravenous tube 3, is prevented.

The peristaltic pump 4 can optionally be mounted on the intravenous tube 3 in order to provide a controlled flow of intravenous fluid. Hereby the rescue personnel can administrate the amount of intravenous fluid delivered to a patient according to instructions from a doctor situated at remote location from the scene of accident. The doctor could e.g. be situated at a hospital and send his instructions to the rescue personnel treating a patient in the field via radio or telephone. Additionally the peristaltic pump 4 is capable of measuring the amount of intravenous fluid delivered to a receiving patient, and providing an alarm signal when the source of intravenous fluid 9 is empty. The peristaltic pump 4 further comprises flow measuring means in order to detect if the flow of intravenous fluid is interrupted, thereby providing an alarm signal if the flow of intravenous fluid is interrupted, e.g. if the intravenous tube 3 is twisted or pinched during transport of the patient. If the peristaltic pump 4 is not used, the alternative intravenous tube 14 shown in fig. 4 could be used instead of the intravenous tube 3.

In a second preferred embodiment of the present invention, the external unit 2 and the peristaltic pump 4 is housed within the same device. Hence, a compact portable device is provided, and when combined with the intravenous tube 3 is capable of delivering a heated and controlled flow of intravenous fluid to a receiving patient. Further it will be possible for the temperature controller to adapt the amount of power supplied to the electrical heating wires 11 according to the actual flow rate of intravenous fluid in the intravenous tube 3, and thus prolonging the operation time of the batteries.

The invention has been exemplified above with reference to specific examples of a system for heating intravenous fluids 1. However, it should be understood that the invention is not limited to the particular examples described above but may be designed and altered in a multitude of varieties within the scope of the invention as specified in the claims.

## Claims

1. A system for heating a flow of intravenous fluid comprising:
an intravenous tube having a liquid inlet and a liquid outlet for connecting a source of intravenous fluid to an intravenous catheter, said intravenous tube comprising heating means integrated therein for raising the temperature of the intravenous flow in the tube, and connection means for connecting said heating means with an external energy source, and
an external unit comprising the external energy source and activating means for activating the heating means in the intravenous tube.

2. A system according to claim 1, wherein said intravenous tube is enclosed in a sterile packaging until time of use.

3. A system according to any of the preceding claims, wherein the surface area in contact with the intravenous fluid of said heating means comprises an area of at least 20 cm², preferably at least 30 cm² and most preferred at least 40 cm².

4. A system according to any of the preceding claims, wherein said integrated heating means comprises one or more heating wires inlaid in the enclosure of the intravenous tube.

5. A system according to any of the preceding claims, wherein said integrated heating means comprising heating wires encased in the wall of the intravenous tube.

6. A system for heating a flow of intravenous fluid according to any of the preceding claims, wherein said heating means extends a substantial portion of the tube length, preferably between 75 cm ando 200 cm of the tube length, preferably between 125 cm and 175 cm of the tube length.

7. A system according to any of the preceding claims, wherein said energy source comprises means for supplying electrical power to the heating means through said connection means.

8. A system according to any of the preceding claims, wherein said connection means and said heating means constitutes a common unit forming a part of the intravenous tube.

9. A system according to claim 8, wherein said external unit comprises an opening for insertion of the common unit, and said opening includes the activating means for supplying said common unit with energy.

10. A system according to claim 9, wherein said activating means comprises electrical connection means, and said common unit comprises an electrical heating element.

11. A system according to claim 9, wherein said activating means comprises an electrical heating element formed in the external unit along a perimeter of said opening, and said common unit comprises a heat exchanger designed to fit into said opening in the external unit.

12. A system according to any of the preceding claims comprising flow control means, such as an pump, for controlling the flow rate of intravenous fluid delivered to a patient.

13. A system according to claim 12, wherein said flow control means comprises a peristaltic pump.

14. A system for heating a flow of intravenous fluid according to any of the preceding claims, wherein said activating means comprises control means for controlling said heating means and/or said means for controlling the flow rate of intravenous fluid delivered to a patient according to at least one control input from means for providing a measure of at least one physical property of the flow of intravenous fluid.

15. A system according to claim 14, wherein said means for providing a measure of at least one physical property of the intravenous fluid comprises means for providing a measure of the flow rate of fluid in the intravenous tube and providing an input accordingly to the control means.

16. A system according to claim 14 or 15, wherein said means for providing a measure of at least one physical property of the intravenous fluid comprises temperature measure means for measuring the temperature of the flow of fluid in the intravenous tube and providing an output accordingly to the control means.

17. A system according to claim 15 or 16, wherein said means for providing a measure of the flow rate of fluid in the intravenous tube comprises alarm means for providing an alarm signal when the source of intravenous fluid is empty.

18. A system according to any of claims 15-17, wherein said means for providing a measure of the flow rate of fluid in the intravenous tube comprises alarm means for providing an alarm signal in case the flow of intravenous fluid is interrupted.

19. A system for heating a flow of intravenous fluid according to any of the preceding claims, wherein said heating means are capable of raising the temperature of the intravenous fluid between 20 °C and 30 °C, preferably between 23 °C and 27 °C at a flow rate of 100 ml/min.

20. A system for heating a flow of intravenous fluid according to any of the preceding claims, comprising means for limiting the temperature of the flow of intravenous fluid to a temperature interval between 35°C and 40°C such as between 36°C and 38°C independently of the flow rate of the intravenous fluid.

21. A system according to any of the preceding claims, wherein said activating means comprises means for automatically transferring energy from said external energy source to said heating means via said connecting means when said connection means is connected to the energy source.

22. Use of a system according to any of claims 1 to 21 for heating a flow of intravenous fluid from a source of intravenous fluid to a catheter inserted into a patient.

23. An assembly for connecting a source of intravenous fluid to a patient, the assembly comprising a sealed sterile packaging enclosing
an intravenous tube having a liquid inlet and a liquid outlet for connecting said source of intravenous fluid to an intravenous catheter, said intravenous tube comprising heating means integrated therein for raising the temperature of the flow of intravenous fluid in the intravenous tube and connection means for connecting said heating means with an external energy source, wherein the mentioned items enclosed in the packaging are sterile.

24. An assembly for connecting a source of intravenous fluid to a patient according to claim 23, wherein said intravenous tube comprises one or more of the technical features of the intravenous tube disclosed in claims 3-10.

25. Heating means for heating a flow of intravenous fluid, comprising
a length of a tube with a fluid connection defined therein,
an electrical heating element integrated within said length of tube, and
electrical connecting means for connecting said heating element to an electrical power source external to said heating means.

26. A length of tube for heating liquid flowing therein comprising
an inner opening with a substantially rectangular cross section,
at least one sheet of electrical heating foil arranged in the longitudinal direction of said rectangular inner opening,
connection means for connecting said electrical heating foil with an external energy source and/or external control means.

27. A length of tube according to claim 26 comprising two sheets of electrical heating foil arranged along the opposing longer sides of the rectangular inner portion.

28. A length of tube according to claim 27, wherein said two sheets of electrical heating foil are short circuited at one end of the tube and are connected to a circuit at the other end for supplying said electrical heating foil with electrical power.

29. A length of tube according to any of claims 26-28, wherein said inner opening with a substantially rectangular cross section has a width in the range of 2 to 6 millimetres, preferably 3 to 5 millimetres, and a millimetres and a length of the longer sides in the range of 4 to 10 millimetres, preferably in the range of 5 to 8 millimetres.

30. A length of tube according to any of claims 26-29, wherein the width of the inner opening constitutes in the range of 30% to 80%, preferably in the range of 40% to 65% of the length of the longer sides of the inner opening.
